Europäisches Patentamt

European Patent Office

Office européen des brevets

(11) Publication number: **0 171 967**
A2

(12) **EUROPEAN PATENT APPLICATION**

(21) Application number: 85305399.9

(22) Date of filing: 29.07.85

(51) Int. Cl.⁴: **A 61 B 17/39**

(30) Priority: 15.08.84 US 640856

(43) Date of publication of application:
19.02.86 Bulletin 86/8

(84) Designated Contracting States:
DE GB IT NL SE

(71) Applicant: VALLEYLAB, INC.
5920 Longbow Drive
Boulder Colorado 80301(US)

(72) Inventor: Grover, Thomas P.
2275 Garland Street
Lakewood Colorado(US)

(74) Representative: Boydell, John Christopher et al,
Stevens, Hewlett & Perkins 5 Quality Court Chancery
Lane
London, WC2A 1HZ(GB)

(54) Electrosurgical generator.

(57) An electrosurgical generator (10) comprises a source of electrosurgical current and output electrode circuitry (12) having an active electrode (22) for applying the electrosurgical current to a patient (26) and a return electrode (24) for returning the current to the source (18), and isolating circuitry for electrically isolating signal processing circuitry connected between the output electrode circuitry (12) and a reference potential such as ground (36) where the isolating circuitry includes an optical power supply (46) for optically powering the signal processing circuit; and an optical output coupler for optically coupling output signals for the signal processing circuit to the electrosurgical current source. The signal processing circuitry may include a handpiece switching circuit and/or a return electrode monitor circuit. Improvements in the foregoing circuits are also disclosed.

FIG. I

Croydon Printing Company Ltd

"ELECTROSURGICAL GENERATOR"

This invention relates to electrosurgical generators and in particular to improve circuitry for optically isolating output electrode connected circuits in such generators.

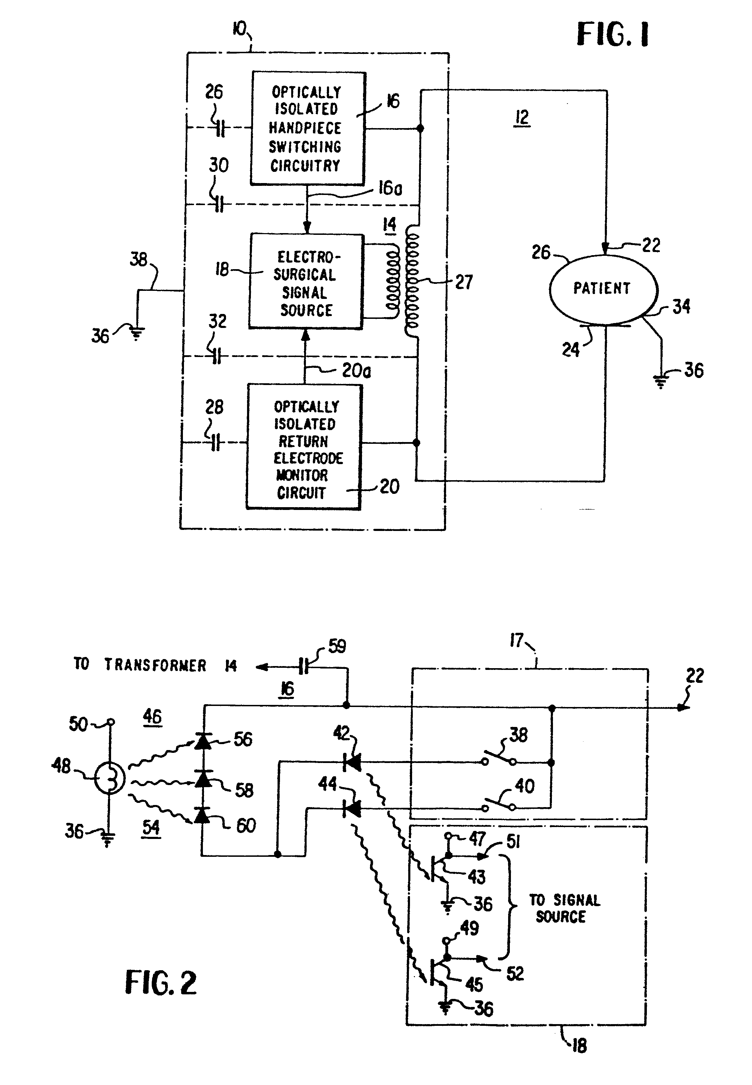

Referring to Fig. 1, there is illustrated an electrosurgical generator coupled to output electrode circuitry 12 through an output transformer 14 where generator 10 may include handpiece switching circuitry 16, an electrosurgical signal source 18, and return electrode monitor circuitry 20 and where output electrode circuitry 12 may include a handpiece having an active electrode 22 and a return electrode 24 connected to a patient 26.  Thus electrosurgical current from source 18 passes from the high side of the secondary winding 27 of output transformer 14 through the active electrode and the patient to the return electrode where it is returned to the low side of the secondary winding.  Electrosurgical action such as cutting or coagulation occurs at the active electrode. The size of the return electrode is such that the current density thereat is reduced to a physiologically benign value.

Typically provided at the handpiece is a switch (not shown but incorporated in switching circuitry 16) which enables the user to determine whether cutting or coagulation will occur at the active electrode. Switching circuitry 16 is responsive to the switch position to thereby apply over line 16a a control signal to cause source 18 to deliver either cut or coagulation current to the handpiece.  Such circuitry is described in U.S. Patent Nos. 3,699,967 and 3,801,800, both of which are incorporated herein by reference.

It is also known to connect monitoring circuitry to the return electrode to ascertain whether the patient is properly connected to the electrode. If the patient is improperly connected, an alarm signal may be applied over line 20a to disable source 18. It is also known to employ as a return electrode either a single-piece element or a two-piece element where the two-piece element is typically referred to as a split return electrode. Circuitry for monitoring the continuity of single- or two-piece return electrodes with respect to the patient is disclosed in U.S. Patent Nos. 4,200,104; 4,416,276; and 4,416,277, all of which are incorporated herein by reference.

A common feature of the handpiece switching circuitry and return electrode monitor circuitry described above is that they are connected to output electrode circuitry 12 as can be seen in Fig. 1. Thus, leakage currents from the output electrode circuitry through these circuits to ground can occur due to leakage capacitance 26 and 28 from the circuits to ground. Furthermore, leakage current from the secondary winding 27 of the output transformer can also occur due to leakage capacitance between the secondary and ground. One-half of the latter capacitance is indicated at 30 and the other half at 32. All these sources of leakage capacitance are potentially harful in that a patient undergoing electrosurgical therapy may become connected to ground 36 via a small area contact such as that indicated at 34 where the small area contact could be effected at electrodes, supports, thermometers or similar equipment connected to the patient. Electrosurgical currents could then flow in the circuit comprising (a) output electrode circuit 12, (b) contact point 34, (c) the wire 38 which grounds the generator (where the phantom line indicates the generator chassis), (d) the leakage capacitance 26-32, and (e) the circuits 16 or 20 back to electrode electrode circuitry 12. Sufficiently large currents may cause burns at contact point 34.

In order to mitigate the foregoing problem, attempts have been made to electrically isolate handpiece switching circuitry 16 and return electrode monitor circuitry 20 from ground 36 and thus decrease the leakage capacitance to decrease the leakage current, it being this leakage current which flows when a patient in contact with a small area contact point 34. In particular, the above-cited patients disclose circuitry for isolating circuits 16 and 20. Thus, in U.S. Patent No. 4,200,104, there is disclosed in Fig. 2 thereof a monitor circuit which monitors a split return electrode, the monitor circuit being isolated from ground. In particular, a power transformer is employed to isolate the circuit with respect to the power supply therefor while the alarm signal therefrom is optically coupled to the electrosurgical signal source, this optical coupling being in lieu of line 16a. However, it has been determined in accordance with the present invention the leakage capacitance through the power transformer is of such a size that current flowing therethrough could be further reduced in the interest of patient safety.

Moreover, in U.S. Patent No. 3,801,800 there is disclosed handpiece switching circuitry isolated from ground. Again, a power transformer is employed to isolate the circuit with respect to the power supply therefor while the control signal therefrom is optically coupled to the electrosurgical signal source, this optical coupling being in lieu of line 20a in a manner similar to that described above for U.S. Patent No. 4,200,104. Again, this circuit is subject to the shortcoming discussed above in that the leakage capacitance through the power transformer is of such a size that current flowing therethrough could be further reduced in the interest of patient safety.

The present invention further mitigates the above-discussed problems of the prior art.

This invention also provides optical coupling for power as well as data in connection with circuits such as handpiece switching circuitry and return electrode monitor circuitry which are connected to the output electrode circuitry of an electrosurgical generator.

Figure 1 is a block diagram of illustrative circuitry in accordance with the present invention which diagram is also utilized to illustrate problems of the prior art.

Figure 2 is a schematic diagram of an illustrative, optically isolated handpiece switching circuit in accordance with the invention.

Figure 3 is a schematic diagram of an illustrative, optically isolated, return electrode monitor circuit in accordance with the invention.

Reference should now be made to the drawing where like reference numerals in the Figures thereof refer to like parts.

Referring to Figure 1, an illustrative embodiment of a system in accordance with the invention is illustrated, the invention being particularly characterized by the use of optical coupling for power as well as data in optically isolated handpiece switching circuitry 16 and optically isolated return electrode monitor circuitry 20. This feature will now be described with respect to Figure 2 which is directed to an optically isolated handpiece switching circuit in accordance with the invention.

In Figure 2, the handpiece switching circuitry 16 includes switches 38 and 40 which are disposed on handpiece 17 in a known manner. Switches 38 and 40 are respectively in series circuit with light emitting diodes (LED's) 42 and 44. The LED's are powered by power source 46 which includes a lamp 48 connected to a voltage supply 50, which typically may be 12 volts, the lamp being grounded at 36. The lamp illuminates an array 54 of elements 56-60 capable of converting light to electric current. Typical of such elements are semiconductor junction devices known as solar cells. Although three cells are illustrated, it is to be underststood the number of cells comprising the array may be varied depending upon the particular application. The area of the array typically comprises a 1 inch x 2 inch area. The open circuit voltage of the array is typically 1.5 volts while the short circuit current is 12 milliamps in sunlight and 6 milliamps with a 2.5 watt lamp.

The LED's 42 and 44 are respectively optically coupled to phototransistors 43 and 45 which are, in turn, respectively connected to power supply terminals 47 and 49 at the collectors thereof and ground 36 at the emitters thereof. The value of the power supply voltage at the terminals may, for example, be 5 volts. The output terminals 51 and 52 are applied to the signal source of electrosurgical signal source 18 in a known manner to thereby cause source 18 to deliver either cut or coagulation current to output electrode circuitry 12 depending on which of the switches 38 or 40 is actuated. Handpiece 17 is also connected to the high side of secondary winding 27 of transformer 14 via capacitor 59 which prevents the circulation of DC current in this line.

In operation, assuming switch 38 is closed to thereby indicate a desire on the part of the user to deliver a cut-type current to active electrode 22, DC current circulates from solar cell array 54 through switch 38 and LED 42 to thus direct a light beam to phototransistor 43. A control signal from terminal 51 is then applied to the signal source to thereby cause the cut-type signal to be delivered through capacitor 59 and thence to active electrode 22 of handpiece 17. As can be appreciated from Figure 2, handpiece switching circuit 16 is straight forward and economical. In prior art switching circuits such as that disclosed in U.S. Patent No. 3,801,800, resistors are normally employed in series with the LED's for current-limiting purposes. However, solar cell aray 54 is self-limiting and, as stated above, delivers only 6 milliamps into a short circuit when illuminated by a 2.5 watt lamp. Hence, the need for the resistors of the prior art is eliminated. Further, by eliminating the power transformer employed in handpiece switching circuitry such as that employed in U.S. Patent No. 3,801,800, substantial economies are possible. Thus, not only is the power transformer eliminated by the circuitry of the present invention but also it associated leakage capacitance, driving circuitry and output filtering. In this regard it should be noted that several solar cells can be driven by a single lamp 48.

With respect to the reduction in leakage capacitance, it shoud be noted the leakage capacitance 26 associated with the handpiece switching circuitry of U.S. Patent No. 3,801,800 is about 15 pf. The total leakage capacitance (that is, capacitances 30 and 32 of Figure 1) associated with output transformer 14 is typically about 20 pf where capacitances 30 and 32 are each about 10 pf. Thus, with respect to the high side of the secondary winding 27, the leakage capacitance is about 25 pf where 15 pf is associated with capacitance 26 and 10 pf is associated with capacitance 30. The total capacitance of 25 pf can carry about 180 milliamps at typical output voltages in an electrosurgical generator. By using optical coupling to power handpiece switching circuit 16 in accordance with the invention, the leakage current is reduced to about 70 milliamps. Hence, the potential for a burn to occur at a contact point such as point 34 is substantially lessened with respect to the prior art. It is also noted the power available from the power source 46 of the present invention is about one quarter of that available from the power source utilized in U.S. Patent No. 3,801,800. Nevertheless, the power available from source 46 is more than enough for solid keying.

It should be further noted the use of solar cells for high voltage isolated power is described in the literature; however, the use of such cells in connection with circuitry such as circuits 16 and 20 to reduce generator output coupling to earthe is new and, in view of the substantial improvement in patient safety, particularly advantageous.

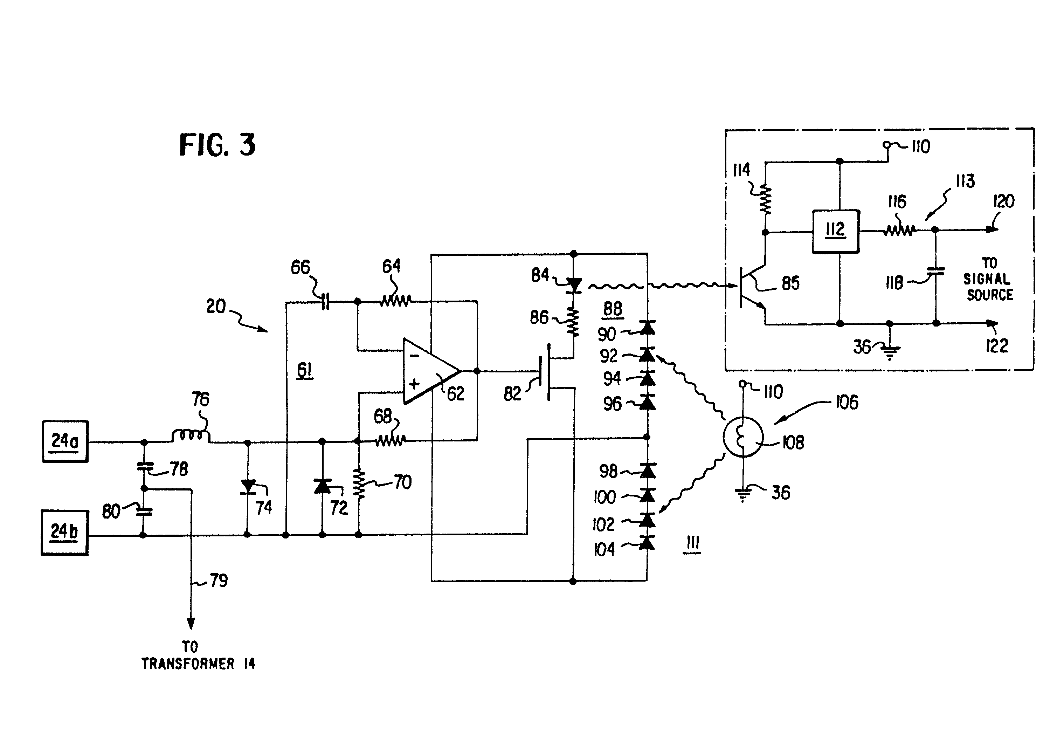

Referring to Figure 3, there is shown an illustrative, optically isolated return electrode monitor circuit 20 in accordance with the invention. The circuit comprises an oscillator 61 which is employed in conjunction with a split return electrode including elements 24a and 24b. The operating frequency of the oscillator varies in accordance with the resistance of the patient disposed between elements 24a and 24b. The output of the oscillator is applied to an LED 84 via an FET 82. The optical output of the LED is applied to a phototransistor 85 and thence to the signal source of electrosurgical source 18 to disable it (or at least provide an alarm signal) if the patient is improperly connected to the split return electrode. The oscillator 61 and LED 84 are powered by a power source 111 in a manner similar to the powering of the handpiece switching circuit 16 described above.

Oscillator 61 includes an operational amplifier 62 having feedback resistors 64 and 68. A capacitor 66 is connected between resistor 64 and return electrode element 24b. A resistor 70 and oppositely-poled diodes 72 and 74 are connected between resistor 68 and element 24b. A radio frequency filter 76 is connected between element 24a and resistor 68 to reduce the effect of electrosurgical current flowing through capacitors 78 and 80 on monitor circuit 20. Line 79 is connected from the point between capacitors 78 and 80 to the low side of secondary winding 27, this connection corresponding to that shown in U.S. Patent No. 4,416,276.

The output of oscillator 61 is applied to FET 82 which is in series with LED 84 and a resistor 86. The circuitry is powered by power source 111 which may include a solar cell array 88 comprising series connected cells 90 through 104. The cells are energized by a light source 106 which may include a lamp 108 connected between a voltage supply terminal 110, which may be 5 volts, and ground 36.

The optical output of LED 84 is applied to phototransistor 85. In particular, the optical output is converted to a voltage by a single shot circuit 112 and a low pass filter 113. The single shot is connected to voltage supply terminal 110 and includes a resistor 114 in circuit therewith. The output of the single shot is applied to low pass filter 113 which includes a resistor 116 and a capacitor 118. The voltage developed by low pass filter 113 may be further processsed by circuitry such as shown in Figure 3 of U.S. Patent No. 4,200,104 to provide an appropriate alarm signal(s) to the signal source of electrosurgical signal source 18, the signal from filter 113 being developed across terminals 120 and 122.

In operation, return electrode monitor circuit 20 is powered by power source 111 where the output power from solar cell array 88 may generally correspond to that described above for array 54. There are many circuits this power source cannot operate; however, due to the simplicity of the return electrode monitor of the present invention, it can be effectively and reliably operated by the solar cell power source of this invention. The frequency of oscillator 61 varies with the patient resistance across elements 24a and 24b. Accordingly, the frequency of the signal coupled from LED 84 to phototransistor 85 varies in the same manner, as does the frequency of the pulse train from signal shot 112. Accordingly, the magnitude of the voltage developed across low pass filter 113 will be proportional to the frequency of the output pulse train from signal shot 112. There is thus developed a signal which may be utilized to disable electrosurgical generator 18 or provide an alarm signal if the patient is improperly connected to return electrode elements 24a and 24b.

When a power transformer is employed to isolate the return electrode monitor circuits such as in U.S. Patent No. 4,200,104, the leakage capacitance 28 is typically 15 pf. Hence, the total leakage capacitance from the low side of the secondary winding 27 to ground is about 25 pf where the leakage capacitance 32 due to the output transformer is 10 pf. Hence, when the power for return electrode monitor circuit 20 is optically coupled in accordance with the present invention, there is a reduction in leakage current corresponding to that discussed above with respect to handpiece switching circuitry 16 - that is, the leakage current is reduced from about 180 milliamps to about 70 milliamps. Thus, with optically coupled circuits on both the active and return ends of the patient connected circuits 16 and 20, the only remaining substantial earth coupling is in rf transformer 14 and accordingly the total leakage impedance due to leakage capacitance 26 through 32 is doubled. This, of course, reduces the risk of patient burn.

Values for the various elements of the monitor circuit of Figure 3 are given below, these values being illustrative and not limitative of the invention:

        resistor 64-100K
        capacitor 66-0.1
        resistor 68-1K
        resistor 70-200
        coil 76-2.5
        capacitors 78 and 80-0.47 each
        resistor 86-1.5K
        resistor 114-10K
        resistor 116-100K
        capacitor 118-1

The above values are in ohms for resistance, microfarads for capacitance and millihenries for inductance.

In general, handpiece switching circuit 16 and return electrode monitor circuit 20 may be considered signal processing circuits. The present invention nearly eliminates the leakage capacitance of these circuits where the input power to each circuit is supplied optically with an incandescent buld or the like illuminating a semiconductor junction light-to-current converter such as a solar cell array. Output from the circuits is from a device such as an LED and is sensed by a device such as a phototransistor. Not only is leakage capacitance minimized, but also the ability to withstand high voltages is greatly increased. Moreover, the resulting circuitry of handpiece switching circuit 16 is extremely simple, where it may use the solar cell junction capacitance for radio frequency filtering and the solar cells' current limited output to eliminate resistors. The return electrode monitor circuit 20 is substantially simpler than that described in U.S. Patent No. 4,416,276; however, it is not as accurate. However, its accuracy is sufficient for many, if not all, applications.

## CLAIMS

1.    An electrosurgical generator (10) including a
source of electrosurgical current and output electrode
circuitry (12) having an active electrode (22) for
applying the electrosurgical current to a patient (26)
and a return electrode (24) for returning the current
to the source (18), characterized by increasing the
electrical isolation of at least one signal processing
circuit connected between the output electrode
circuitry (12) and a reference potential comprising
        optical power supply means (46) for optically
    powering said signal processing circuit; and
        optical output means for optically coupling
    output signals from said signal processing circuit
    to said electrosurgical current source.

2.    A generator according to Claim 1
characterized in that said reference potential is
ground (36).

3.    A generator according to Claim 1
characterized in that said signal processing circuit
includes at least two switches (38, 40) disposed on a
handpiece (17) for said active electrode, where said
optical output means includes a pair of output elements
(42, 44) respectively associated with said switches
where each of said output elements emits an output
light signal in response to the flow of electrical
current therethrough, and where said optical power
supply means (46) includes at least one light sensitive
element (48) for generating the electrical current for
said output elements, said light sensitive element
being responsive to input light to convert it to said
electrical current, and a source of said input light.

4.    A generator according to Claim 3 characterized in that said light sensitive element comprises a semiconductor junction light-to-current convertor.

5.    A generator according to Claim 3 characterized in that said light sensitive element comprises a solar cell.

6.    A generator according to Claim 3 characterized by having a plurality of said light sensitive elements.

7.    A generator according to Claim 3 characterized in that said electrosurgical current source includes a pair of further light sensitive elements (56, 58, 60) respectively responsive to the output light signals emitted by said pair of output elements (42, 44) to thereby generate respective control signals for said electrosurgical current source indicating which of said pair of switches is closed to thus establish the nature of the electrosurgical current applied to the patient.

8.    A generator according to any one of Claims 1 to 7, characterized in that said signal processing circuit comprises monitor circuitry (20) for monitoring the continuity of said patient with respect to said return electrode and for modifying the operation of the electrosurgical current source depending on the degree of continuity of the patient with the return electrode (24).

9.    A generator according to Claim 8 characterized in that said return electrode (24) includes two separated elements and said monitor circuitry (20) includes a variable frequency oscillator (61), the frequency of which is a function of the resistance of the patient between the two elements (24a, 24b) of the return electrode and the output of which is applied to an output element (84) which emits an output light signal which is function of the oscillator output, said oscillator and said output element being powered by electrical currents supplied thereto.

10. A generator according to Claim 9 characterized in that said monitor circuitry (20) includes at least one light sensitive element for generating said electrical currents for said oscillator (61) and output element (84), said light sensitive element being responsive to input light to convert it to said electrical currents, and a source of said input light.

0171967

FIG. 1

FIG. 2

FIG. 3